(19) Europäisches Patentamt
European Patent Office
Office européen des brevets

(11) EP 0 748 807 A1

(12) # DEMANDE DE BREVET EUROPEEN

(43) Date de publication:
**18.12.1996 Bulletin 1996/51**

(51) Int Cl.[6]: **C07D 471/08**, A61K 31/495

(21) Numéro de dépôt: **96401249.6**

(22) Date de dépôt: **11.06.1996**

(84) Etats contractants désignés:
**AT BE CH DE DK ES FI FR GB GR IE IT LI LU NL PT SE**

(30) Priorité: **13.06.1995 FR 9506951**

(71) Demandeur: **SYNTHELABO**
**F-92350 Le Plessis Robinson (FR)**

(72) Inventeurs:
- **Jegham, Samir**
  **95100 Argenteuil (FR)**
- **Koenig, Jean Jacques**
  **78600 Maisons Laffitte (FR)**
- **Lochead, Alistair**
  **94220 Charenton le Pont (FR)**
- **Nedelec, Alain**
  **92700 Colombes (FR)**
- **Guminski, Yves**
  **81090 La Garrigue (FR)**

(74) Mandataire: **Ludwig, Jacques et al**
**SYNTHELABO,**
**Service Brevets,**
**B.P. 72**
**92352 Le Plessis Robinson Cédex (FR)**

(54) **Dérivés de N- (1,4-diazabicyclo 2.2.2 oct-2-yl)méthyl benzamide, leur préparation et leur application en thérapeutique**

(57) Composés répondant à la formule générale (I)

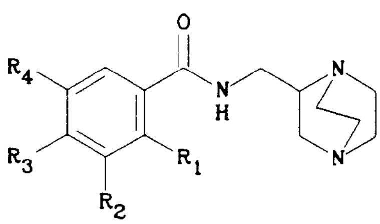

(I)

dans laquelle $R_1$ représente un groupe méthoxy ou cyclopropylméthoxy, $R_2$ représente un atome d'hydrogène, de chlore ou de brome ou bien encore $R_1$ et $R_2$ forment ensemble, un groupe de formule $-C-CH_2-O-$, $-O-(CH_2)_2-$ , $-O-(CH_2)_2-O-$ ou $-O-(CH_2)_3-O-$, $R_3$ représente un atome d'hydrogène ou un groupe amino, et $R_4$ représente un atome d'hydrogène, de chlore ou de brome.

Application en thérapeutique.

EP 0 748 807 A1

## Description

La présente invention a pour objet des dérivés de *N*-[(1,4-diazabicyclo[2.2.2]oct-2-yl)méthyl]benzamide, leur préparation et leur application en thérapeutique.

Les composés de l'invention répondent à la formule générale (I)

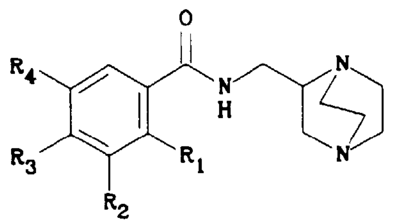

(I)

dans laquelle

$R_1$ représente un groupe méthoxy ou cyclopropylméthoxy,

$R_2$ représente un atome d'hydrogène, de chlore ou de brome, ou bien encore

$R_1$ et $R_2$ forment ensemble, et dans cet ordre, un groupe de formule $-O-CH_2-O-$, $-O-(CH_2)_2-$, $-O-(CH_2)_2-O-$ ou $-O-(CH_2)_3-O-$,

$R_3$ représente un atome d'hydrogène ou un groupe amino, et

$R_4$ représente un atome d'hydrogène, de chlore ou de brome.

Ils peuvent exister à l'état de bases libres ou de sels d'addition à des acides. Par ailleurs ils comportent, dans le cycle de diazaoctane, un atome de carbone asymétrique, et peuvent donc exister sous forme d'énantiomères purs ou de mélanges d'énantiomères.

Les composés les plus intéressants sont, en général, les composés de formule générale (I) dans laquelle $R_3$ représente un groupe amino et $R_4$ représente un atome de chlore.

Parmi les composés préférés on peut citer le (+)-8-amino-7-chloro-*N*-[(1,4-diazabicyclo[2.2.2]oct-2-yl)méthyl]-2,3-dihydro-1,4-benzodioxine-5-carboxamide, le (-)-8-amino-7-chloro-*N*-[(1,4-diazabicyclo[2.2.2]oct-2-yl)méthyl]-2,3-dihydro-1,4-benzodioxine-5-carboxamide, le (+)-4-amino-5-chloro-2-(cyclopropylméthoxy)-*N*-[(1,4-diazabicyclo[2.2.2]oct-2-yl)méthyl]benzamide, le (-)-4-amino-5-chloro -2-(cyclopropylméthoxy)-*N*-[(1,4-diazabicyclo[2.2.2]oct-2-yl)méthyl]benzamide, le (-)-8-amino-7-bromo-*N*-[(1,4-diazabicyclo[2.2.2]oct-2-yl)méthyl]-2,3-dihydro-1,4-benzodioxine-5-carboxamide, le (-)-9-amino-8-chloro-*N*-[(1,4-diazabicyclo[2.2.2]oct-2-yl)méthyl]-3,4-dihydro-2*H*-1,5-benzodioxépine-6-carboxamide.

Conformément à l'invention on peut préparer les composés de formule générale (I) par un procédé illustré par le schéma qui suit.

On fait réagir un aminométhyloxirane protégé, de formule (II), dans laquelle Pht représente un groupe phtalimido (ou 1,3-dihydro-1,3-dioxo-2*H*-isoindol-2-yle), avec une pipérazine protégée de formule (III), dans laquelle Bn représente un groupe benzyle, à une température de 80 à 120°C, en l'absence de solvant ou dans un solvant aprotique, par exemple le toluène ou le dioxane.

On obtient l'alcool de formule (IV), qu'on fait réagir avec le chlorure de méthanesulfonyle en présence de triéthylamine, dans un solvant aprotique, par exemple le dichlorométhane, à une température de -5 à +20°C, puis on chauffe le composé obtenu, de formule (V), à une température de 110°C pendant 2 à 8 h, dans un solvant aprotique, par exemple le toluène, pour obtenir l'ammonium quaternaire de formule (VI).

On débenzyle ce dernier par hydrogénation catalytique en présence de charbon palladié pour obtenir le composé de formule (VII), puis on déprotège ce dernier au moyen d'hydrate d'hydrazine dans un solvant alcoolique, par exemple l'éthanol, à la température du reflux pendant 1 à 3 h.

On obtient l'amine de formule (VIII), dont on prépare un sel d'addition, par exemple le trichlorhydrate.

Finalement on fait réagir ce composé avec un acide de formule générale (IX), dans laquelle $R_1$, $R_2$, $R_3$ et $R_4$ sont tels que définis ci-dessus, en présence de dicyclohexylcarbodiimide, dans un solvant aprotique, par exemple la pyridine, à température ambiante.

Schéma

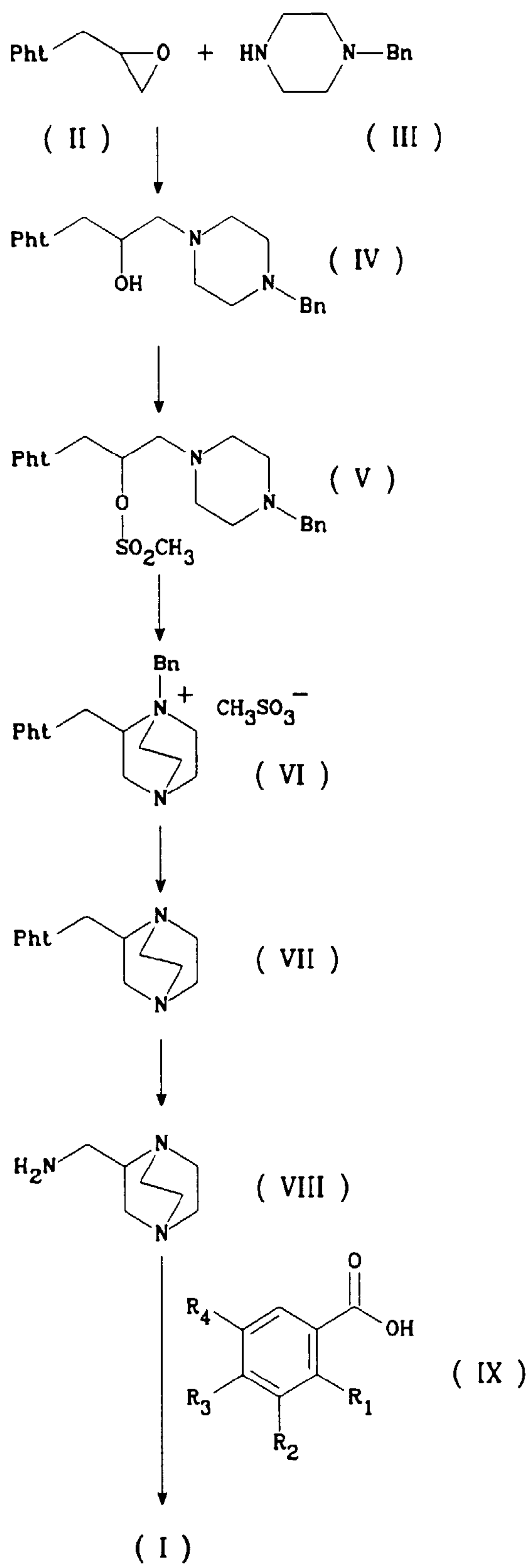

( II ) ( III )

( IV )

( V )

( VI )

( VII )

( VIII )

( IX )

( I )

Le composé de départ de formule (II) peut être obtenu à partir de 4-méthylbenzènesulfonate d'oxiranylméthyle et du sel de potassium de la 1*H*-isoindole-1,3(2*H*)-dione, disponibles dans le commerce, dans le toluène et en présence d'un agent de transfert de phase tel que le bromure de hexadécyltributylphosphonium. La structure stéréochimique du tosylate détermine celle du composé final : le tosylate racémique permet la préparation d'un composé final racémique, et un tosylate optiquement pur permet la préparation d'un composé final optiquement pur.

Un autre procédé de préparation du composé de formule (II), à partir de l'un ou de l'autre énantiomère du 2,2-diméthyl-1,3-dioxolane-4-méthanol, tous deux disponibles dans le commerce, est illustré dans le détail par l'exemple 4 qui suit. Il consiste à faire réagir l'un des énantiomères du 2,2-diméthyl-1,3-dioxolane-4-méthanol avec le chlorure de méthanesulfonyle pour obtenir le méthanesulfonate de (2,2-diméthyl-1,3-dioxolan-4-yl)méthyle correspondant, puis à traiter ce dernier avec du phtalimidure de potassium en présence d'un agent de transfert de phase tel que le bromure de hexadécyltributylphosphonium, pour obtenir la [(2,2-diméthyl-1,3-dioxolan-4-yl)méthyl]-1*H*-isoindole-1,3(2*H*)-dione correspondante, puis à traiter cette dernière avec de l'acide chlorhydrique dilué pour obtenir la 2-(2,3-dihydroxypropyl)-1*H*-isoindole-1,3(2*H*)-dione correspondante, puis à faire réagir cette dernière avec le benzaldéhyde, pour obtenir la [(2-phényl-1,3-dioxolan-4-yl)méthyle]-1*H*-iso-indole-1,3(2*H*)-dione correspondante, puis à faire réagir cette dernière avec le *N*-bromosuccinimide pour obtenir le benzoate de 2-bromométhyl-1-(1,3-dihydro-1,3-dioxo-2*H*-iso-indol-2-yl) éthyle correspondant, et enfin on traite ce dernier avec le méthylate de sodium pour provoquer la cyclisation en 2-(oxiranylméthyl)-1H-isoindole-1,3(2H)-dione optiquement pure.

La benzylpipérazine de formule (III) est disponible dans le commerce.

Certains acides de formule générale (IX) sont disponibles dans le commerce ; les autres peuvent être préparés par des méthodes telles que celles décrites dans *J. Med. Chem.* (1993), **36**, 4121-4123 et dans les demandes de brevets EP-0234872, WO-9305038 et ES-2019042, ou par saponification d'esters correspondants tels que ceux décrits dans les brevets DE-3001328 et DE-36433103.

Les exemples suivants illustrent en détail la préparation de quelques composés selon l'invention. Les microanalyses élémentaires et les spectres IR et RMN confirment les structures des composés obtenus.
Les numéros des composés indiqués entre parenthèses dans les titres correspondent à ceux du tableau donné plus loin.

<u>Exemple 1</u> (Composé N°1)

Dichlorhydrate de (±)-4-amino-5-chloro-*N*-[(1,4-diazabicyclo-[2.2.2]oct-2-yl)méthyl]-2-méthoxybenzamide.

1.1. 2-[2-Hydroxy-3-[4-(phénylméthyl)pipérazin-1-yl]propyl]-1*H*-isoindol-1,3(2*H*)-dione.

On chauffe 34,7 g (0,197 mole) de 1-(phénylméthyl)pipérazine à 80°C, on ajoute, par petites portions, 40,0 g (0,197 mole) de 2-(oxiranylméthyl)-1*H*-isoindol-1,3(2*H*)-dione, et on chauffe le mélange à 100°C pendant 5 min.
On le laisse refroidir, on le reprend avec 50 ml de toluène, on ajoute 350 ml d'heptane et on obtient une huile qui cristallise en refroidissant.
Après filtration et séchage des cristaux on obtient 71,2 g de composé.
Point de fusion : 94°C.

1.2. Méthanesulfonate de (±)-2-(1,3-dioxo-1,3-dihydro-2*H*-isoindol-2-yl)-1-[[4-(phénylméthyl)pipérazin-1-yl]méthyl] éthyle.

On refroidit à 0°C une solution de 59,8 g (0,158 mole) de 2-[2-hydroxy-3-[4-(phénylméthyl)pipérazin-1-yl]propyl]-1*H*-isoindol-1,3(2*H*)-dione dans 350 ml de dichlorométhane, on ajoute 19,1 g (0,189 mole) de triéthylamine puis, goutte à goutte, 19,8 g (0,173 mole) de chlorure de méthanesulfonyle. Après 1 h on ajoute 400 ml d'eau, on sépare la phase organique, on évapore le solvant sous pression réduite et on purifie le résidu par chromatographie sur colonne de gel de silice en éluant avec un mélange d'acétate d'éthyle et d'heptane 90/10.
On obtient 62,3 g de composé qu'on utilise tel quel dans l'étape suivante.

1.3. Méthanesulfonate de (±)-2-(1,3-dioxo-1,3-dihydro-2*H*-isoindol-2-yl)méthyl-1-(phénylméthyl)-4-aza-1-azoniabicyclo[2.2.2]octane.

On chauffe pendant 14 h à la température du reflux une solution de 61 g (0,133 mole) de méthanesulfonate de 2-(1,3-dioxo-1,3-dihydro-2*H*-isoindol-2-yl)-1-[[4-(phénylméthyl)pipérazin-1-yl]méthyl]éthyle dans 500 ml de toluène, on refroidit le mélange, on sépare le solide par filtration, on le rince avec du toluène et on le sèche.
On obtient 52,9 g de composé qu'on utilise tel quel dans l'étape suivante.
Point de fusion : >260°C.

1.4. (±)-2-[(1,4-Diazabicyclo[2.2.2]oct-2-yl)méthyl]-2*H*-isoindole-1,3(2*H*)-dione.

On place dans un hydrogénateur une solution de 52,6 g (0,115 mole) de méthanesulfonate de (±)-2-(1,3-dioxo-1,3-dihydro-2*H*-isoindol-2-yl)méthyl-1-(phénylméthyl)-4-aza-1-azoniabicyclo[2.2.2]octane dans 500 ml de méthanol, on ajoute 15 g de charbon palladié à 10%, et on effectue une hydrogénation catalytique à 50°C sous 0,1 MPa pendant 1 h.
On sépare le catalyseur par filtration, en le rinçant avec du méthanol, et on évapore le filtrat sous pression réduite. On obtient 21,1 g de composé.
Point de fusion : 156°C.

1.5. Trichlorhydrate de (±)-1,4-diazabicyclo[2.2.2]octane-2-méthanamine.

On chauffe un mélange de 20,0 g (0,0737 mole) de 2-[(1,4-diazabicyclo[2.2.2]oct-2-yl)méthyl]-2*H*-isoindole-1,3(2*H*)-dione et 4,4 g (0,0884 mole) d'hydrate d'hydrazine dans 200 ml d'éthanol au reflux pendant 2 h 30 min.
On concentre le mélange, on reprend le résidu avec 300 ml de chloroforme, on élimine l'insoluble par filtration, on évapore le filtrat sous pression réduite et on purifie le résidu sur une colonne d'alumine en éluant avec un mélange de chloroforme, de méthanol et d'ammoniaque 90/10/1. On obtient 9,3 g de liquide jaune qu'on distille sous pression réduite. On obtient ainsi 8,45 g de liquide incolore. Point d'ébullition : 70°C sous 100 Pa.
On prépare le trichlorhydrate en dissolvant 8,25 g (0,0584 mole) de ce composé dans 50 ml d'éthanol à 10°C, on ajoute 50 ml d'une solution 4N d'acide chlorhydrique dans l'éthanol, jusqu'à pH = 1, on refroidit la solution à 0°C et on filtre le sel qui précipite. Après séchage sous pression réduite on obtient 14,3 g de trichlorhydrate.
Point de fusion : 280°C.

1.6. Dichlorhydrate de (±)-4-amino-5-chloro-*N*-[(1,4-diazabicyclo[2.2.2]oct-2-yl)méthyl]-2-méthoxybenzamide.

On ajoute 1,25 g (0,005 mole) de trichlorhydrate de 1,4-diazabicyclo[2.2.2]octane-2-méthanamine à une solution de 0,2 g (0,005 mole) de soude dans 1,6 ml d'eau, on ajoute, goutte à goutte, une solution de 1,11 g (0,0055 mole) d'acide 4-amino-5-chloro-2-méthoxybenzoïque dans 6,7 ml de pyridine, puis, en deux fois, et en respectant un intervalle de temps de 45 min, on élimine l'insoluble par filtration, on concentre le filtrat, on ajoute 1,86 g (0,009 mole) de dicyclohexylcarbodiimide, et on maintient l'agitation à température ambiante pendant une nuit.
On ajoute 15 ml d'eau, on agite pendant 45 min, on filtre le mélange et on évapore le filtrat sous pression réduite. On reprend le résidu avec de l'eau, on ajoute de la soude aqueuse jusqu'à pH = 10, on recueille le précipité par filtration et on le purifie par chromatographie sur colonne de gel de silice en éluant avec un mélange de de chloroforme, de méthanol et d'ammoniaque 85/15/1,5. On obtient 1,28 g de produit qu'on purifie en le triturant à chaud dans 20 ml d'éthanol. Après filtration, évaporation et séchage à 50°C sous pression réduite, on obtient finalement 1,2 g de composé sous forme de base. Point de fusion : 232°C.
On en met 1,0 g (0,00308 mole) en suspension dans 10 ml d'éthanol, on ajoute 0,7 ml (0,008 mmole) d'acide chlorhydrique concentré puis 2 ml d'eau, on filtre la solution obtenue et on la concentre à sec. On reprend le résidu avec de l'éthanol et on concentre de nouveau à sec. On répète l'opération, puis on sèche le produit sous pression réduite. On obtient finalement 0,93 g de dichlorhydrate.
Point de fusion : 239°C.

Exemple 2 (Composé N°2)

Dichlorhydrate de (+)-4-amino-5-chloro-*N*-[(1,4-diazabicyclo[2.2.2]oct-2-yl)méthyl]-2-méthoxybenzamide.

2.1. (-)-2-(Oxiranylméthyl)-1*H*-isoindole-1,3(2*H*)-dione.

On chauffe un mélange de 40,75 g (0,22 mole) de phtalimidure de potassium, 5,07 g (0,01 mole) de bromure de hexadécyltributylphosphonium et 160 ml de toluène à 80°C.
On introduit, en 30 min, 45,6 g (0,2 mole) de (+)-4-méthylbenzènesulfonate d'oxiranylméthyle en solution dans 80 ml de toluène et on maintient le chauffage entre 80 et 100°C pendant 2 h.
On filtre le mélange, on lave le filtrat quatre fois à l'eau, on le sèche, on évapore le solvant sous pression réduite, on fait cristalliser le résidu dans un mélange d'acétate d'éthyle et d'éther diisopropylique 1/2 et on le purifie par chromatographie sur colonne de gel de silice en éluant avec un mélange d'acétate d'éthyle et d'heptane 50/50.
On obtient 28 g de produit qu'on triture dans l'éther diisopropylique. On obtient 24,9 g de composé.
Point de fusion : 100°C.
$[\alpha]_D^{20}$ = -6° (c = 1 ; $CHCl_3$).

2.2. Trichlorhydrate de (+)-1,4-diazabicyclo[2.2.2]octane-2-méthanamine.

On procède comme décrit dans les exemples 1.2 à 1.5, mais à partir de la (-)-2-(oxiranylméthyl)-1*H*-isoindole-1,3 (2*H*)-dione optiquement pure préparée à l'étape précédente.
Point de fusion : 240°C (décomposition).
$[\alpha]_D^{20}$ = +20,7° (c = 1 ; $H_2O$).

2.3. Dichlorhydrate de (+)-4-amino-5-chloro-*N*-[(1,4-diazabicyclo[2.2.2]oct-2-yl)méthyl]-2-méthoxybenzamide.

On procède comme décrit dans l'exemple 1.6, mais à partir du trichlorhydrate de (+)-1,4-diazabicyclo[2.2.2]octane-2-méthanamine optiquement pure préparée à l'étape précédente.
Point de fusion : 228°C (décomposition).
$[\alpha]_D^{20}$ = +14,4° (c = 1 ; $H_2O$).

Exemple 3 (Composé N°3)

Dichlorhydrate de (-)-4-amino-5-chloro-*N*-[(1,4-diazabicyclo[2.2.2]oct-2-yl)méthyl]-2-méthoxybenzamide.

3.1. (+)-2-(Oxiranylméthyl)-1*H*-isoindole-1,3(2*H*)-dione.

On procède comme décrit dans l'exemple 2.1, mais à partir de (-)-4-méthylbenzènesulfonate d'oxiranylméthyle.
Point de fusion : 97°C.
$[\alpha]_D^{20}$ = +8,5° (c = 1 ; $CH_2Cl_2$).

3.2. Trichlorhydrate de (-)-1,4-diazabicyclo[2.2.2]octane-2-méthanamine.

On procède comme décrit dans les exemples 1.2 à 1.5, mais à partir de la (+)-2-(oxiranylméthyl)-1*H*-isoindole-1,3 (2*H*)-dione optiquement pure préparée à l'étape précédente.
Point de fusion : 240°C (décomposition).
$[\alpha]_D^{20}$ = -22,9° (c = 1 ; $H_2O$).

3.3. Dichlorhydrate de (-)-4-amino-5-chloro-*N*-[(1,4-diazabicyclo[2.2.2]oct-2-yl)méthyl]-2-méthoxybenzamide.

On procède comme décrit dans l'exemple 1.6, mais à partir du trichlorhydrate de (-)-1,4-diazabicyclo[2.2.2]octane-2-méthanamine optiquement pure préparée à l'étape précédente.
Point de fusion : 215°C (décomposition).
$[\alpha]_D^{20}$ = -12,7° (c = 1 ; $H_2O$).

Exemple 4 (Composé N°8)

Dichlorhydrate de (+)-8-amine-7-chloro-*N*-[(1,4-diazabicyclo[2.2.2]oct-2-yl)méthyl]-2,3-dihydro-1,4-benzodioxine-5-carboxamide.

4.1. Méthanesulfonate de (-)-(2,2-diméthyl-1,3-dioxolan-4-yl)méthyle.

A une solution de 300 g (2,269 moles) de (-)-2,2-diméthyl-1,3-dioxolane-4-méthanol dans 2,05 l de dichlorométhane on ajoute 380 ml (2,723 moles) de triéthylamine, on refroidit la solution à -5°C et on ajoute, goutte à goutte et pendant 1h10, 193 ml (2,496 moles) de chlorure de méthanesulfonyle. Après 10 min d'agitation on lave la solution quatre fois à l'eau, on la sèche et on évapore le solvant sous pression réduite.
On obtient 453 g de produit liquide orange qu'on utilise tel quel dans l'étape suivante.

4.2. (+)-2-[(2,2-Diméthyl-1,3-dioxolan-4-yl)méthyl]-1*H*-isoindole-1,3(2*H*)-dione.

On chauffe à 60°C un mélange de 398,9 g (2,154 moles) de sel de potassium de la 1*H*-isoindole-1,3(2*H*)-dione et 109,3 g (0,215 mole) de bromure de hexadécyltributylphosphonium dans 3,25 l de toluène, puis on ajoute, goutte à goutte et en 20 min, 453 g (2,154 moles) de méthanesulfonate de (-)-(2,2-diméthyl-1,3-dioxolan-4-yl)méthyle et on chauffe le mélange à 80°C pendant 4h.
On ajoute encore 54,7 g (0,108 mole) de bromure de hexadécyltributylphosphonium et on poursuit le chauffage entre

80 et 100°C pendant 5h.
On refroidit le mélange vers 60°C, on le lave avec de l'eau puis deux fois avec une solution de chlorure de sodium, on sèche la phase organique et on évapore le solvant sous pression réduite.
On obtient 691,6 g de produit solide orange.
Point de fusion : 81,9-82°C.
$[\alpha]_D^{20}$ = +35,2° (c = 1 ; $CH_2Cl_2$).

4.3. (+)-2-(2,3-Dihydroxypropyl)-1*H*-isoindole-1,3(2*H*)-dione.

On chauffe une suspension de 261,3 g (2,154 moles) de (+)-[(2,2-diméthyl-1,3-dioxolan-4-yl)méthyl]-1*H*-isoindole-1,3(2*H*)-dione, 2,8 l d'eau et 5,6 ml d'acide chlorhydrique 12M à 60°C pendant 2h.
On neutralise le milieu avec 11 ml d'hydroxyde de sodium aqueux 10M et on évapore 800 ml d'eau.
On refroidit le résidu à 12°C et on obtient 352,9 g de solide blanc.
Point de fusion : 122,5-122,8°C.
$[\alpha]_D^{20}$ = +48,4° (c = 1 ; $CH_3OH$).
$[\alpha]_{365}^{20}$ = 210,8° (c = 1 ; $CH_3OH$).

4.4. (+)-2-[(2-Phényl-1,3-dioxolan-4-yl)méthyle]-1*H*-isoindole-1,3(2*H*)-dione.

On chauffe au reflux un mélange de 22,1 g (0,1 mole) de (*R*)-(+)-2-(2,3-dihydroxypropyl)-1*H*-isoindole-1,3(2*H*)-dione, 10,6 g (0,1 mole) de benzaldéhyde, 100 ml de toluène et 0,1 g d'acide paratoluènesulfonique pendant 3h.
On refroidit le mélange, on le lave à l'eau puis avec une solution aqueuse d'hydrogénocarbonate de sodium, on sèche la phase organique, on évapore le solvant sous pression réduite et on fait cristalliser le résidu dans 100 ml d'un mélange 20/80 d'éther diéthylique et d'éther diisopropylique.
On obtient 25,9 g d'un mélange 50/50 de diastéréoisomères.
Point de fusion : 84°C.
$[\alpha]_D^{20}$ = +62° (c = 1 ; $CH_2Cl_2$).

4.5. Benzoate de (+)-2-bromométhyl-1-(1,3-dihydro-1,3-dioxo-2*H*-isoindol-2-yl)éthyle.

A une solution de 19,4 g (0,0627 mole) de (+)-2-[(2-phényl-1,3-dioxolan-4-yl)méthyle]-1*H*-isoindole-1,3(2*H*)-dione dans 100 ml de 1,2-dichloroéthane on ajoute, par petites portions, 11,2 g (0,0627 mole) de *N*-bromosuccinimide et on agite le mélange pendant 2h.
On filtre le mélange, on lave le filtrat avec de l'eau contenant du thiosulfate de sodium, on sèche la phase organique, on évapore le solvant sous pression réduite et on triture le résidu dans de l'éther diéthylique.
On obtient 28 g de produit.
Point de fusion : 120°C.
$[\alpha]_D^{20}$ = +57,5° (c = 1 ; $CH_2Cl_2$).

4.6. (-)-2-(Oxiranylméthyl)-1*H*-isoindole-1,3(2*H*)-dione.

On prépare une solution de méthylate de sodium à partir de 1,17 g (0,051 mole) de sodium et 15 ml de méthanol, et, en 20 min et à 30°C, on l'ajoute à une solution de 19,7 g (0,0507 mole) de benzoate de (+)-2-bromométhyl-1-(1,3-dihydro-1,3-dioxo-2*H*-isoindol-2-yl)éthyle dans 200 ml de toluène, et on agite le mélange à température ambiante pendant une nuit.
On lave la solution à l'eau, on évapore le solvant sous pression réduite et on triture le résidu dans un mélange d'éther diisopropylique et d'heptane.
On obtient 9,7 g de produit.
Point de fusion : 101,4-101,5°C.
$[\alpha]_D^{20}$ = -6,6° (c = 1 ; $CHCl_3$).
$[\alpha]_{365}^{20}$ = -48,5° (c = 1 ; $CHCl_3$).

4.7. Dichlorhydrate de (+)-8-amino-7-chloro-*N*-[(1,4-diazabicyclo[2.2.2]oct-2-yl)méthyl]-2,3-dihydro-1,4-benzodioxine-5-carboxamide.

On procède comme décrit dans l'exemple 1, à partir de (-)-2-(oxiranylméthyl)-1*H*-isoindole-1,3(2*H*)-dione optiquement pure.
Point de fusion : 231°C (décomposition).

$[\alpha]_D^{20}$ = +17,8° (c = 1 ; $H_2O$).

Exemple 5 (Composé N°9)

Dichlorhydrate de (-)-8-amino-7-chloro-*N*-[(1,4-diazabicyclo[2.2.2]oct-2-yl)méthyl]-2,3-dihydro-1,4-benzodioxine-5-carboxamide.

5.1. Méthanesulfonate de (+)-(2,2-diméthyl-1,3-dioxolan-4-yl)méthyle.

On procède comme décrit dans l'exemple 4.1, mais à partir de (+)-2,2-diméthyl-1,3-dioxolane-4-méthanol.

5.2. (-)-2-[(2,2-Diméthyl-1,3-dioxolan-4-yl)méthyl]-1*H*-isoindole-1,3(2*H*)-dione.

On procède comme décrit dans l'exemple 4.2, à partir de méthanesulfonate de (+)-(2,2-diméthyl-1,3-dioxolan-4-yl) méthyle.
Point de fusion : 81,2-81,3°C.
$[\alpha]_D^{20}$ = -34,9° (c = 1 ; $CH_2Cl_2$).

5.3. (-)-2-(2,3-Dihydroxypropyl)-1*H*-isoindole-1,3(2*H*)-dione.

On procède comme décrit dans l'exemple 4.3, à partir de (-)-2-[(2,2-diméthyl-1,3-dioxolan-4-yl)méthyl]-1*H*-isoindole-1,3(2*H*)-dione.
Point de fusion : 122,8-122,9°C.
$[\alpha]_D^{20}$ = -48,8° (c = 1 ; $CH_3OH$).

5.4. (-)-2-[(2-Phényl-1,3-dioxolan-4-yl)méthyle]-1*H*-isoindole-1,3(2*H*)-dione.

On procède comme décrit dans l'exemple 4.4, à partir de (-)-2-(2,3-dihydroxypropyl)-1*H*-isoindole-1,3(2*H*)-dione.
Point de fusion : 84°C.
$[\alpha]_D^{20}$ = -59° (c = 1 ; $CH_2Cl_2$).

5.5. Benzoate de (-)-2-bromométhyl-1-(1,3-dihydro-1,3-dioxo-2*H*-isoindol-2-yl)éthyle.

On procède comme décrit dans l'exemple 4.5, à partir de (-)-2-[(2-phényl-1,3-dioxolan-4-yl)méthyle]-1*H*-isoindole-1,3(2*H*)-dione.
Point de fusion : 118,4-118,6°C.
$[\alpha]_D^{20}$ = -58,2° (c = 1 ; $CH_2Cl_2$).

5.6. (+)-2-(Oxiranylméthyl)-1*H*-isoindole-1,3(2*H*)-dione. Point de fusion :

On procède comme décrit dans l'exemple 4.6, à partir de benzoate de (-)-2-bromométhyl-1-(1,3-dihydro-1,3-dioxo-2*H*-isoindol-2-yl)éthyle.
Point de fusion : 100,4-100,5°C.°C.
$[\alpha]_{365}^{20}$= +45,5° (c = 1 ; $CHCl_3$).

5.7. Dichlorhydrate de (-)-8-amino-7-chloro-*N*-[(1,4-diazabicyclo[2.2.2]oct-2-yl)méthyl]-2,3-dihydro-1,4-benzodioxine-5-carboxamide.

On procède comme décrit dans l'exemple 4.7, à partir de (+)-2-(oxiranylméthyl)-1*H*-isoindole-1,3(2*H*)-dione.
Point de fusion : 220°C (décomposition).
$[\alpha]_D^{20}$ = -16,9° (c = 1 ; $H_2O$).

Exemple 6 (Composé N°20)

Dichlorhydrate de (-)-6-chloro-*N*-[(1,4-diazabicyclo[2.2.2]oct-2-yl)méthyl]-1,3-benzodioxolane-4-carboxamide.

6.1. Acide 6-chloro-1,3-benzodioxolane-4-carboxylique.

On prépare une suspension de 5,0 g (30,1 mmoles) d'acide 1,3-benzodioxolane-4-carboxylique dans 50 ml d'acide acétique, on la chauffe à 70°C, on ajoute, en 15 min, 1,0 g de *N*-chlorosuccinimide, on poursuit le chauffage pendant 30 min, on ajoute de nouveau 1,0 g de *N*-chlorosuccinimide, et, après encore 15 min de chauffage, on ajoute 2,0 g de *N*-chlorosuccinimide, soit un total de 4,0 g (30,1 mmoles) et on maintient le chauffage à 70°C pendant 2 h.
On laisse refroidir le mélange, on le verse sur 150 ml d'eau, on collecte le solide par filtration, on le lave à l'eau et on le sèche sous vide.
On obtient 1,72 g de solide qu'on purifie par chromatographie sur colonne de gel de silice en éluant avec un mélange 95/5/0,5 de dichlorométhane, méthanol et acide acétique.
Après recristallisation dans un mélange d'éthanol et d'eau on obtient 1,13 g de composé.
Point de fusion : 210°C.

6.2. Dichlorhydrate de (-)-6-chloro-*N*-[(1,4-diazabicyclo[2.2.2]oct-2-yl)méthyl]-1,3-benzodioxolane-4-carboxamide.

On effectue la réaction avec la (-)-1,4-diazabicyclo[2.2.2]_ octane-2-méthanamine comme décrit précédemment et on prépare le dichlorhydrate du composé obtenu.
Point de fusion : 212°C.
$[\alpha]_D^{20}$ = -18° (c = 1 ; $H_2O$).

Le tableau qui suit illustre les structures chimiques et les propriétés physiques de quelques composés selon l'invention.
Dans la colonne "$R_1$", $cC_3H_5$ désigne un groupe cyclopropyle.
Dans la colonne "Sel", "-" désigne un composé à l'état de base libre et 2HCl désigne un dichlorhydrate.
Dans la colonne "F (°C)", (d) désigne un point de fusion avec décomposition.
Dans la colonne "$[\alpha]_D^{20}$", le pouvoir rotatoire est déterminé à c = 1 ; $H_2O$ pour les dichlorhydrates, et à c = 1 ; $CH_3OH$ pour les bases (composés n°12, 13 et 19). La valeur 0 désigne un racémate.

Tableau

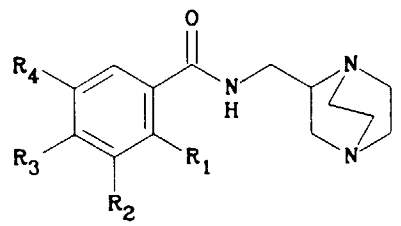

(I)

| N° | $R_1$ | $R_2$ | $R_3$ | $R_4$ | Sel | F (°C) | $[\alpha]_D^{20}$ |
|---|---|---|---|---|---|---|---|
| 1 | $-OCH_3$ | H | $NH_2$ | Cl | 2HCl | 239 | 0 |
| 2 | $-OCH_3$ | H | $NH_2$ | Cl | 2HCl | 228 (d) | +14,4 |
| 3 | $-OCH_3$ | H | $NH_2$ | Cl | 2HCl | 215 | -12,7 |
| 4 | $-OCH_3$ | H | H | Cl | 2HCl | 223 | +12,7 |
| 5 | $-OCH_3$ | H | $NH_2$ | H | 2HCl | 210-235 (d) | +8,7 |
| 6 | $-O-(CH_2)_2-$ | | H | Cl | 2HCl | 197-250 (d) | +8,1 |
| 7 | $-O-(CH_2)_2-$ | | H | Cl | 2HCl | 229-265 | -13,8 |
| 8 | $-O-(CH_2)_2-O-$ | | $NH_2$ | Cl | 2HCl | 231 (d) | +17,8 |
| 9 | $-O-(CH_2)_2-O-$ | | $NH_2$ | Cl | 2HCl | 220 (d) | -16,9 |
| 10 | $-OCH_2cC_3H_5$ | H | $NH_2$ | Cl | 2HCl | 210-215 (d) | +7,9 |
| 11 | $-OCH_2cC_3H_5$ | H | $NH_2$ | Cl | 2HCl | 220-230 | -12,0 |
| 12 | $-OCH_3$ | Cl | $NH_2$ | Cl | - | 212-255 | +23,5 |
| 13 | $-OCH_3$ | Cl | $NH_2$ | Cl | - | 212-255 | -22,8 |
| 14 | $-OCH_3$ | Br | $NH_2$ | Br | 2HCl | 230 | +12,5 |
| 15 | $-OCH_3$ | Br | $NH_2$ | Br | 2HCl | 195 | -10,8 |
| 16 | $-O-(CH_2)_2-O-$ | | H | Cl | 2HCl | 253-254 | +20,0 |
| 17 | $-O-(CH_2)_2-O-$ | | H | Cl | 2HCl | 220 | -18,5 |
| 18 | $-O-(CH_2)_2-O-$ | | $NH_2$ | Br | 2HCl | 226-228 | -16,5 |
| 19 | $-O-(CH_2)_3-O-$ | | $NH_2$ | Cl | - | 214 | -24,6 |
| 20 | $-O-CH_2-O-$ | | H | Cl | 2HCl | 212 | -18,0 |

Les composés de l'invention ont fait l'objet d'essais qui ont mis en évidence leur intérêt comme substances à activités thérapeutiques.

On a ainsi mis en évidence leur affinité pour les récepteurs sérotoninergiques 5-$HT_3$ par le déplacement de la

liaison d'un ligand spécifique marqué, le [$^3$H]-(*S*)-Zacopride.
L'étude est effectuées *in vitro* sur les récepteurs 5-$HT_3$ du cortex de rat, essentiellement comme décrit par Barnes N. M. et coll., *J. Pharm. Pharmacol.* (1988) **40** 548-551.
Des rats mâles Sprague-Dawley (OFA, Iffa Credo, Lyon, France), d'un poids de 200 à 250 g, sont euthanasiés et le cerveau est prélevé. Ensuite le cortex est disséqué et homogénéisé à l'aide d'un broyeur Polytron™ (position 7,20 s) dans 20 volumes de tampon Tris (25 mM, ph = 7,4 à 22°C), l'homogénat est centrifugé à 45000 g pendant 10 min à l'aide d'une centrifugeuse Sorvall™ munie d'un rotor SS34, le culot est remis en suspension dans 10 volumes de tampon Tris et incubé à 37°C pendant 10 min sous agitation.
La suspension est diluée à 20 volumes a l'aide de tampon Tris, et centrifugée de nouveau dans les mêmes conditions, puis le culot est remis en suspension dans 5 volumes de tampon Tris et réparti en fractions aliquotes de 5 ml qui sont congelées à -80°C.
Le jour de l'expérience, la préparation est décongelée à +4°C puis diluée 1,2 fois à l'aide du tampon d'incubation Tris-NaCl (Tris 25 mM, NaCl 150 mM, pH = 7,4 à 22°C).
La suspension membranaire (100 µl, 1 mg de protéines) est alors incubée à 25°C pendant 25 min en présence de 0,5 nM de [$^3$H]-(*S*)-Zacopride (activité spécifique 75-85 Ci/mmole, Amersham, Little Chalfont, Grande-Bretagne) dans un volume final de 500 µl de tampon Tris-NaCl en présence ou en absence de composé à tester.
L'incubation est arrêtée par filtration sur filtres Whatman GF/B préalablement traités avec de la polyéthylèneimine à 0,1%. Chaque tube réactionnel est prédilué avec 4 ml de tampon Tris-NaCl puis rincé trois fois avec 4,5 ml de de tampon Tris-NaCl.
Les filtres sont prédécoupés avant séchage dans l'étuve (120°C, 5 min). La radioactivité retenue sur les filtres est déterminée par scintigraphie liquide. La liaison non spécifique est déterminée en présence de 10 µM de MDL 72222 (ligand décrit dans l'article cité).
Pour chaque concentration de composé étudié on détermine le pourcentage d'inhibition de la liaison spécifique du [$^3$H]-(*S*)-Zacopride, puis la concentration $CI_{50}$, concentration de ce composé qui inhibe 50% de cette liaison spécifique.
Les $CI_{50}$ des composés de l'invention se situent entre 0,009 et 1 µM.

Les composés de l'invention ont été également étudiés quant à leur affinité vis-à-vis des récepteurs 5-$HT_4$ dans le striatum de cobaye selon la méthode décrite par Grossman et coll. dans *Br.* J. *Pharmacol.* (1993) **109** 618-624.
On euthanasie des cobayes (Hartley, Charles River, France) de 300 à 400 g, on prélève les cerveaux, on excise les striata et on les congèle à -80°C.
Le jour de l'expérience on décongèle le tissu à +4°C dans 33 volumes de tampon HEPES-NaOH (50 mM, pH = 7,4 à 20°C), on l'homogénéise à l'aide d'un broyeur Polytron™, on centrifuge l'homogénat à 48000 g pendant 10 min, on récupère le culot, on le remet en suspension, on le centrifuge de nouveau dans les mêmes conditions et on remet le culot final en suspension dans du tampon HEPES-NaOH, à raison de 30 mg de tissu par ml.
On fait incuber 100 µl de cette suspension membranaire à 0°C pendant 120 min en présence de [$^3$H]GR113808 (ligand décrit dans l'article cité, activité spécifique 80-85 Ci/mmole) dans un volume final de 1 ml de tampon HEPES-NaOH (50 mM, pH = 7,4), en présence ou en absence de composé à tester. On arrête l'incubation par filtration sur filtre Whatman GF/B préalablement traité avec de la polyéthylèneimine à 0,1%, on rince chaque tube avec 4 ml de tampon à 0°C, on filtre de nouveau et on mesure la radioactivité retenue sur le filtre par scintigraphie liquide.
On détermine la liaison non spécifique en présence de sérotonine 30 µM. La liaison spécifique représente 90% de la radioactivité totale récupérée sur le filtre.
Pour chaque concentration de composé étudié on détermine le pourcentage d'inhibition de la liaison spécifique du [$^3$H] GR113808 puis la $CI_{50}$, concentration du composé testé qui inhibe 50% de la liaison spécifique.
Les $CI_{50}$ des composés de l'invention se situent entre 0,008 et 1 µM.

Les composés de l'invention ont aussi été étudiés quant à leurs effets agonistes ou antagonistes vis-à-vis des récepteurs 5-$HT_4$ dans l'oesophage de rat selon la méthode décrite par Baxter et coll. dans *Naunyn Schmied. Arch. Pharmacol.* (1991) **343** 439.
On utilise des rats mâles Sprague-Dawley pesant de 300 à 450 g. On prélève rapidement un fragment d'environ 1,5 cm de la partie terminale de l'oesophage, on élimine la couche musculaire, on ouvre longitudinalement la tunique muqueuse musculaire interne, on la monte dans une cuve à organe isolé contenant une solution de Krebs-Henseleit à 32°C oxygénée par un courant carbogène (95% $O_2$ et 5% $CO_2$), et on la connecte à un transducteur isométrique sous une tension basale de 0,5 g. On induit une contraction du tissu par l'addition de 05 µM de carbachol, on attend que la contraction se stabilise (15 min), puis on expose la préparation à la sérotonine (1 µM) afin de quantifier la relaxation maximale. On lave le tissu et, après une période de 20 min, on ajoute à nouveau 0,5 µM de carbachol, et on expose la préparation au composé à étudier, en dosses cumulées croissantes de 0,1 à 1 µM.
Les composés qui induisent une relaxation sont caractérisés comme des agonistes 5-$HT_4$.
Pour les composés qui n'induisent pas de relaxation, la préparation est exposée à la sérotonine en concentrations cumulées croissantes, de 0,1 nM jusqu'à une concentration induisant une relaxation maximale, et la courbe de relaxation due à la sérotonine, en présence du composé à étudier, est alors comparée à une courbe témoin établie en

l'absence dudit composé. Si sa présence induit un déplacement de la courbe vers la droite, le composé étudié est caractérisé comme un antagoniste 5-$HT_4$.
Enfin les composés de l'invention ont été étudiés quant-à-leurs effets antagonistes vis-à-vis des récepteurs 5-$HT_3$ du muscle lisse de côlon descendant isolé de cobaye, selon la méthode décrite par Grossman et coll. dans *Br.* J. *Pharmacol.* (1989) **97** 451.
La sérotonine (0,1-100 μM), après blocage des récepteurs de types 5-$HT_1$ et 5-$HT_2$ (Methysergide 0,1 μM) et désensibilisation des récepteurs 5-$HT_4$ (5-méthoxytryptamine 10 μM) provoque une contraction, dépendante de la concentration, de la partie musculaire lisse du côlon descendant de cobaye, par stimulation des récepteurs 5-$HT_3$. Les contractions sont enregistrées en isométrie.
L'effet antagoniste d'un composé sur les récepteurs sérotoninergiques 5-$HT_3$ est quantifié par la mesure du déplacement d'une courbe effet-concentration témoin de sérotonine (concentrations successives croissantes non cumulées), à des concentrations du composé comprises entre 1 nM et 0,1 μM, avec une incubation de 30 min.

Les résultats des essais biologiques effectués sur les composés de l'invention montrent qu'ils sont des ligands des récepteurs sérotoninergiques de types 5-$HT_3$ et/ou 5-$HT_4$, et qu'ils agissent comme des agonistes ou antagonistes 5-$HT_4$ et/ou comme des antagonistes 5-$HT_3$.

Les composés peuvent donc être utilisés pour le traitement et la prévention des désordres dans lesquels les récepteurs 5-$HT_3$ et/ou 5-$HT_4$ sont impliqués, que ce soit au niveau du système nerveux central, du système gastro-intestinal, du système cardio-vasculaire ou du système urinaire.

Au niveau du système nerveux central, ces désordres et troubles comprennent notamment les troubles neurologiques et psychiatriques tels que les troubles cognitifs, les psychoses, les comportements compulsifs et obsessionnels et les états de dépression et d'anxiété. Les troubles cognitifs comprennent, par exemple, les déficits de mémoire et d'attention, les états de démence (démences séniles du type de la maladie d'Alzheimer ou démences liées à l'âge), les déficiences cérébrales vasculaires, la maladie de Parkinson. Les psychoses comprennent, par exemple, la paranoïa, la schizophrénie, la manie et l'autisme. Les comportements compulsifs et obsessionnels comprennent, par exemple, les troubles alimentaires du type de la boulimie ou de la perte d'appétit. Les états de dépression et d'anxiété comprennent, par exemple, les anxiétés de type anticipatoire (avant intervention chirurgicale, avant traitement dentaire, etc), l'anxiété causée par la dépendance ou le sevrage d'alcool, de drogue, la manie, les désordres affectifs saisonniers, les migraines, les nausées.

Au niveau du système gastro-intestinal, ces désordres et troubles comprennent notamment les vomissements induits par un traitement antitumoral, les troubles directs ou indirects de la gastromotilité de l'oesophage, de l'estomac ou des intestins, les maladies spécifiques comme la dyspepsie, l'ulcère, le reflux gastro-oesophagien, la flatulence, le syndrome du côlon irritable, les troubles de la sécrétion intestinale, les diarrhées, par exemple celles induites par le choléra ou par le syndrome carcinoïde.

Au niveau du système cardio-vasculaire, ces désordres et troubles comprennent notamment les pathologies liées, directement ou indirectement, aux arythmies cardiaques.

Au niveau du système urinaire, ces désordres et troubles comprennent notamment les incontinences de toutes sortes, ainsi que leurs causes ou conséquences, par exemples les infections, les calculs ou les dommages rénaux.

Les composés de l'invention peuvent être présentés sous toutes formes de compositions appropriées à l'administration entérale ou parentérale, telles que comprimés, dragées, gélules, capsules, suspensions ou solutions buvables ou injectables telles que sirops ou ampoules, etc, associés à des excipients convenables, et dosés pour permettre une administration journalière de 0,005 à 20 mg/kg.

## Revendications

1. Composé, sous forme d'énantiomère pur ou d'un mélange d'énantiomères, répondant à la formule générale (I)

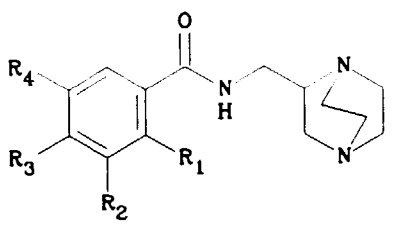

(I)

dans laquelle

$R_1$ représente un groupe méthoxy ou cyclopropylméthoxy,
$R_2$ représente un atome d'hydrogène, de chlore ou de brome, ou bien encore
$R_1$ et $R_2$ forment ensemble, et dans cet ordre, un groupe de formule $-O-CH_2-O-$, $-O-(CH_2)_2-$, $-O-(CH_2)_2-O-$ ou $-O-(CH_2)_3-O-$,
$R_3$ représente un atome d'hydrogène ou un groupe amino, et
$R_4$ représente un atome d'hydrogène, de chlore ou de brome, à l'état de base libre ou de sel d'addition à un acide.

**2.** Composé selon la revendication 1, caractérisé en ce que $R_3$ représente un groupe amino et $R_4$ représente un atome de chlore.

**3.** Composé selon la revendication 1, en l'espèce le (+)-8-amino-7-chloro-*N*-[(1,4-diazabicyclo[2.2.2]oct-2-yl)-méthyl]-2,3-dihydro-1,4-benzodioxine-5-carboxamide, à l'état de base libre ou de sel d'addition à un acide.

**4.** Composé selon la revendication 1, en l'espèce le (-)-8-amino-7-chloro-*N*-[(1,4-diazabicyclo[2.2.2]oct-2-yl)-méthyl]-2,3-dihydro-1,4-benzodioxine-5-carboxamide, à l'état de base libre ou de sel d'addition à un acide.

**5.** Composé selon la revendication 1, en l'espèce le (+)-4-amino-5-chloro -2-(cyclopropylméthoxy)-*N*-[(1,4-diazabicyclo[2.2.2]oct-2-yl)méthyl]benzamide, à l'état de base libre ou de sel d'addition à un acide.

**6.** Composé selon la revendication 1, en l'espèce le (-)-4-amino-5-chloro -2-(cyclopropylméthoxy)-*N*-[(1,4-diazabicyclo[2.2.2]oct-2-yl)méthyl]benzamide, à l'état de base libre ou de sel d'addition à un acide.

**7.** Composé selon la revendication 1, en l'espèce le (-)-8-amino-7-bromo-*N*-[(1,4-diazabicyclo[2.2.2]oct-2-yl)méthyl]-2,3-dihydro-1,4-benzodioxine-5-carboxamide, à l'état de base libre ou de sel d'addition à un acide.

**8.** Composé selon la revendication 1, en l'espèce le (-)-9-amino-8-chloro-*N*-[(1,4-diazabicyclo[2.2.2]oct-2-yl)méthyl]-3,4-dihydro-2*H*-1,5-benzodioxépine-6-carboxamide, à l'état de base libre ou de sel d'addition à un acide.

**9.** Procédé de préparation d'un composé selon la revendication 1, caractérisé en ce qu'on fait réagir un aminométhyloxirane protégé, de formule (II)

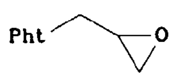

(II)

dans laquelle Pht représente un groupe phtalimido, avec une pipérazine protégée de formule (III)

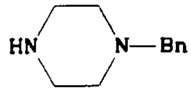

(III)

dans laquelle Bn représente un groupe benzyle, pour obtenir l'alcool de formule (IV)

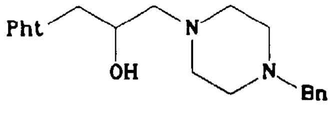

(IV)

qu'on fait réagir avec le chlorure de méthanesulfonyle en présence de triéthylamine, puis on chauffe le composé obtenu, de formule (V)

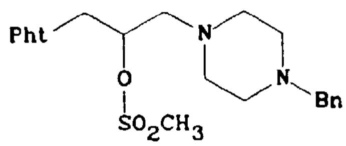

(V)

pour obtenir l'ammonium quaternaire de formule (VI)

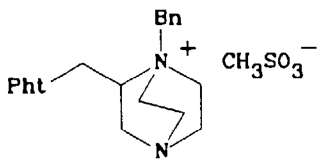

(VI)

puis on débenzyle ce dernier par hydrogénation catalytique pour obtenir le composé de formule (VII)

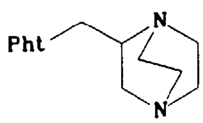

(VII)

puis on déprotège ce dernier au moyen d'hydrate d'hydrazine, pour obtenir l'amine de formule (VIII)

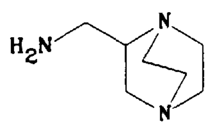

(VIII)

et finalement on fait réagir ce composé avec un acide de formule générale (IX)

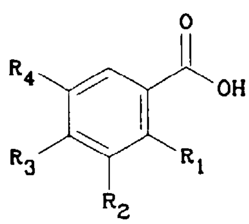

(IX)

dans laquelle $R_1$, $R_2$, $R_3$ et $R_4$ sont tels que définis dans la revendication 1.

**10.** Procédé selon la revendication 9, caractérisé en ce que, pour préparer la 2-(oxiranylméthyl)-1*H*-isoindole-1,3(2*H*)-dione optiquement pure de formule (II), on fait réagir l'un des énantiomères du 2,2-diméthyl-1,3-dioxolane-4-méthanol avec le chlorure de méthanesulfonyle pour obtenir le méthanesulfonate de (2,2-diméthyl-1,3-dioxolan-4-yl) méthyle correspondant, puis on traite ce dernier avec du phtalimidure de potassium pour obtenir la [(2,2-diméthyl-1,3-dioxolan-4-yl)méthyl]-1*H*-isoindole-1,3(2*H*)-dione correspondante, puis on traite cette dernière avec de l'acide chlorhydrique dilué pour obtenir la 2-(2,3-dihydroxypropyl)-1*H*-isoindole-1,3(2*H*)-dione correspondante, puis on fait réagir cette dernière avec le benzaldéhyde, pour obtenir la [(2-phényl-1,3-dioxolan-4-yl)méthyle]-1*H*-isoindole-1,3(2*H*)-dione correspondante, puis on fait réagir cette dernière avec le *N*-bromosuccinimide pour obtenir le benzoate de 2-bromométhyl-1-(1,3-dihydro-1,3-dioxo-2*H*-isoindol-2-yl)-éthyle correspondant, et enfin on traite ce dernier avec le méthylate de sodium pour provoquer la cyclisation en 2-(oxiranylméthyl)-1*H*-isoindole-1,3(2*H*)-dione optiquement pure.

**11.** Médicament caractérisé en ce qu'il consiste en un composé selon la revendication 1.

**12.** Composition pharmaceutique caractérisée en ce qu'elle contient un composé selon la revendication 1, associé à un excipient.

Office européen des brevets

# RAPPORT DE RECHERCHE EUROPEENNE

Numero de la demande
EP 96 40 1249

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,P | WO-A-95 18104 (YAMANOUCHI PHARMA CO LTD ) 6 Juillet 1995<br>* page 41; revendications; exemple 11 *<br>--- | 1-12 | C07D471/08<br>A61K31/495 |
| A,D | WO-A-93 05038 (SMITHKLINE BEECHAM PLC) 18 Mars 1993<br>* le document en entier *<br>--- | 1-12 | |
| A,D | EP-A-0 234 872 (ADRIA LAB INC) 2 Septembre 1987<br>* le document en entier *<br>----- | 1-12 | |
| | | | DOMAINES TECHNIQUES RECHERCHES (Int.Cl.6) |
| | | | C07D<br>A61K |

Le présent rapport a été établi pour toutes les revendications

| Lieu de la recherche | Date d'achèvement de la recherche | Examinateur |
|---|---|---|
| LA HAYE | 1 Juillet 1996 | Bosma, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)